(19) Europäisches Patentamt
European Patent Office
Office européen des brevets

(11) Publication number: 0 256 559
A2

# (12) EUROPEAN PATENT APPLICATION

(21) Application number: 87112030.9

(51) Int. Cl.[4]: C07C 68/06 , C07C 69/96

(22) Date of filing: 19.08.87

(30) Priority: 20.08.86 JP 195027/86

(43) Date of publication of application:
24.02.88 Bulletin 88/08

(84) Designated Contracting States:
CH DE FR LI

(71) Applicant: MITSUBISHI CHEMICAL INDUSTRIES LIMITED
5-2, Marunouchi 2-chome Chiyoda-ku
Tokyo 100(JP)

(72) Inventor: Tamura, Mitsuhiko
9-16, Higashioojimachi Yahatanishi-ku
Kitakyushu-shi Fukuoka-ken(JP)
Inventor: Yamada, Noriichi
2-71-502, 1-ch., Aoyama Yahatanishi-ku
Kitakyushu-shi Fukuoka-ken(JP)
Inventor: Ohta, Masaru
2766-5, Ooaza
Nakama-shi Fukuoka-ken(JP)
Inventor: Yahata, Toshihiko
29-3, 9-chome Hinosato
Munakata-shi Fukuoka-ken(JP)

(74) Representative: Vossius & Partner
Siebertstrasse 4 P.O. Box 86 07 67
D-8000 München 86(DE)

## (54) Process for producing di-tertiary-butyl dicarbonate.

(57) A process for producing di-tert-butyl dicarbonate which comprises reacting a monoalkali metal mono-tert-butyl carbonate with phosgene or trichloromethyl chloroformate in the presence of a tertiary amine in an organic solvent. It provides a higher yield of product and more productive on a commercial scale, because of the shorter reaction step.

EP 0 256 559 A2

Xerox Copy Centre

# PROCESS FOR PRODUCING DI-TERTIARY-BUTYL DICARBONATE

## BACKGROUND OF THE INVENTION

### 1. FIELD OF THE INVENTION

The present invention relates to a process for producing di-tert-butyl dicarbonate (hereinafter referred to as Di-BOC), which is useful as a protecting agent for amino groups and is called Di-BOC as a common name, advantageously on an industrial scale.

### 2. DESCRIPTION OF THE PRIOR ART

Di-BOC is known as a protecting agent for amino groups, which protects various amino groups by tert-butoxy carbonylation of amino group. It is an ideal protecting agent for amino groups, since the said carbonylation reaction can be adequately conducted and the by-products thereof are, for the most part, tert-butanol and carbon dioxide so that the treatment after the reaction is simple.

However, there are problems with respect to the reaction and operation to produce Di-BOC industrially, whereby it has been heretofore impossible to produce Di-BOC at low cost. Therefore, Di-BOC is utilized in laboratories, but is not widely utilized on an industrial scale.

As a method for producing Di-BOC, there has been known, for example, the following equations.

Firstly, alkali metal tert-butoxide reacts with carbon dioxide in an organic solvent such as tetrahydrofuran to form a monocarbonate according to the equation (1),

$$(CH_3)_3COK + CO_2 \longrightarrow (CH_3)_3CO\overset{\overset{O}{\|}}{C}OK \qquad --- (1)$$

which then reacts with phosgene to obtain a tricarbonate according to the equation (2),

$$2(CH_3)_3CO\overset{\overset{O}{\|}}{C}OK + COCl_2 \longrightarrow (CH_3)_3CO\overset{\overset{O}{\|}}{C}O\overset{\overset{O}{\|}}{C}O\overset{\overset{O}{\|}}{C}OC(CH_3)_3 + 2KCl \qquad --- (2)$$

which is then isolated and is contacted with a tertiary amine such as 1,4-diazabicyclo [2,2,2] octane to recover the desired product, i.e. Di-BOC by decarboxylation according to equation (3).

$$(CH_3)_3CO\overset{\overset{O}{\|}}{C}O\overset{\overset{O}{\|}}{C}O\overset{\overset{O}{\|}}{C}OC(CH_3)_3 \longrightarrow \underset{\text{(Di-BOC)}}{(CH_3)_3CO\overset{\overset{O}{\|}}{C}O\overset{\overset{O}{\|}}{C}OC(CH_3)_3} + CO_2 \qquad --- (3)$$

[See, for example, Org. Synth., 57, 45 (1977), Proc. Nat. Acad. Sci. USA, 69, 730 (1972), J. Org. Chem., 35, 3393 (1970)]

However, according to the above-mentioned known method, the process is carried out via tricarbonate obtained by the reaction of equation (2) and the tricarbonate is isolated to supply to the reaction of equation (3), whereby the yield is inadequate and the operation is remarkably complicated and difficult to carry out. Namely, according to the above-mentioned literature, the yield of tricarbonate obtained by the reaction of equation (2) is found to be in the range of 64 - 75%, and furthermore it is required to purify the tricarbonate in order to supply it to the reaction of equation (3), whereby the yield of the purified tricarbonate is found to be in the range of 59 - 62%.

In the reaction mixture obtained by the reaction of equation (2), there are a number of acidic substances such as tert-butyl chloroformate besides unreacted phosgene. Accordingly, even if 1,4-diazabicyclo [2,2,2] octane is added to this reaction mixture, the decarboxylation of equation (3) does not proceed. In the above-mentioned literature, it is disclosed that the reaction of equation (3) does not proceed sufficiently due to a presence of coexisting acidic substances even in case of using the tricarbonate isolated by crystallization in a yield of from 64 to 75% and it is recomended to purity the tricarbonate still more. Furthermore, since the reaction mixture obtained by the reaction of equation (2) shows a paste-like state, as a whole, which includes fine crystals of by-product salt, the separation of the tricarbonate from this paste-like reaction product is remarkably difficult. That is, if the separation could be conducted by a conventional manner such as washing with water since the by-product salt is soluble in water, it would be simple as an industrial operation. However, the separation of the by-product salt should have been conducted by a difficult filtration over a period of many hours, since the tricarbonate was unstable to water.

According to the conventional method, as described above, the operation is remarkably complicated and difficult to carry out, since the process is carried out via the tricarbonate which should be isolated. Further, the yield of tricarbonate is at a low level, whereby the overall yield of Di-BOC is of course inadequate.

## SUMMARY OF THE INVENTION

Now, it has been found that Di-BOC may be directly prepared in high yield by reacting a monocarbonate with phosgene in the presence of a tertiary amine and that such separation step of an intermediate tricarbonate as in the known process is unnecessary. Further, it has been found that even though the reaction mixture is a paste-like material containing Di-BOC and by-product salt, Di-BOC is so stable to water that the reaction mixture can be readily washed with water to remove the by-product salt.

An object of this present invention is to provide an improved process for the preparation of Di-BOC in high yield which is superior to the known process for the reasons set out hereinafter.

The present invention is characterized in that the process for producing Di-BOC by reacting monoalkali metal mono-tert-butyl carbonate with phosgene or trichloromethyl chloroformate in the presence of a tertiary amine in an organic solvent.

## DETAILED DESCRIPTION OF THE PREFERRED EMBODIMENTS

Now, the present invention will be described in detail with reference to the preferred embodiments.

The monoalkali metal mono-tert-butyl carbonate as a starting material in the present invention, can be usually obtained by reacting an alkali metal tert-butoxide with carbon dioxide. This reaction is conducted usually by introducing carbon dioxide into the solution or suspension of alkali metal tert-butoxide dissolved or suspended in an organic solvent at a temperature of not higher than 50°C, preferably from -20° to 30°C, wherein the amount of carbon dioxide introduced is from 0.5 to 10 mols, preferably from 1 to 3 mols relative to 1 mol of alkali metal tert-butoxide. As the organic solvent used here, there may be mentioned, for example, a cyclic ether such as tetrahydrofuran and dioxane, an aliphatic ether such as diethyl ether, isopropyl ether, dibutyl ether and dimethoxy ethane, an aromatic hydrocarbon such as benzene and toluene, an aliphatic hydrocarbon such as hexane and heptane, a halogenated aliphatic hydrocarbon such as methylene chloride and carbon tetrachloride, or a mixture thereof. Upon completion of the reaction, the mixture being paste-like as a whole is obtained wherein the crystals of monoalkali metal mono-tert-butyl carbonate are dispersed in the organic solvent. So, it is desirable to use this mixture as it is as a starting material according to the present invention. Optionally, said crystals of monoalkali metal mono-tert-butylcarbonate which are separated from the mixture may be used. As an alkali metal of the aforesaid monoalkali metal salt, there may be mentioned usually potassium or sodium.

In the present invention, the reaction is conducted by reacting a monoalkali metal mono-tert-butyl monocarbonate with phosgene or trichloromethyl chloroformate in the presence of a tertiary amine in an organic solvent.

Since trichloromethyl chloroformate is readily decomposed into phosgene which reacts with monoalkali metal mono-tert-butyl carbonate in the reaction system, the present invention will be described below in the case of phosgene.

It is usual to use phosgene in an amount of from 0.4 to 5 mols, preferably from 0.5 to 1.5 mols relative to 1 mol of the aforesaid monoalkali metal mono-tert-butyl carbonate. If the amount used is too little, the reaction is conducted inadequately whereby a number of unreacted materials remain in the reaction mixture.

On the contrary, even though the amount used is too much, the better results of this reaction are not given whereby, the cost of phosgene and of making it harmless after the reaction increases, which is uneconomical. Phosgene is used usually in a state of liquid, solution dissolving it in an organic solvent, or gas. When the gaseous phosgene is used, it may be diluted, if desired, with an inert gas such as nitrogen gas or carbondioxide gas.

In the present invention, the reaction is conducted in an organic solvent. As a solvent, there may be mentioned the same as that used in the above-mentioned preparation of the alkali metal tert-butyl carbonate. The organic solvent is used usually in an amount of from 2 to 100 times, preferably from 4 to 20 times as much as the weight of the said monoalkali metal mono-tert-butyl carbonate.

Further, as a tertiary amine which is present in the reaction system in the present invention, there are mentioned usually an aliphatic amine such as trimethylamine, triethylamine and tributylamine, an aromatic amine such as dimethylaniline and diethylaniline, an alicyclic amine such as 1,4-diazabicyclo [2,2,2] octane, 1,8-diazabicyclo [5,4,0] undecene-7, hexamethyltetramine, N-methylpiperidine, N-ethylpiperidine, N-methylmorpholine, N-ethylmorpholine and N,N'-dialkylpiperazine, a heterocyclic amine such as pyridine, quinoline and isoquinoline. Among these amines, triethylamine and 1,4-diazabicylo [2,2,2] octane are preferred. These tertiary amines are used usually in an amount of from 0.005 to 50 mol%, preferably from 0.005 to 5 mol% to the aforesaid monoalkali metal mono-tert-butyl carbonate. If the amount used is too little, the desired product i.e. Di-BOC can not be obtained effectively. Whereas if the amount used is too much, there have no particular effects which is uneconomical.

The reaction temperatures according to the present invention are usually not higher than 100°C, preferably from -20° to 50°C. Remarkably low temperatures are disadvantageous in that a greater cost requires for a cooling equipment. On the other hand, too high temperatures lead to a decomposition of the starting material as well as the product, whereby Di-BOC cannot be obtained in good yield.

The reaction time is in general approximately in the range of 0.5 to 20 hours.

In the present invention, the reaction is conducted usually by adding a predetermined amount of a tertiary amine into an organic solvent which contains monoalkali metal mono-tert-butyl carbonate, followed by supplying gradually phosgene thereto under stirring while externally cooling so that the internal temperatures of the reactor are maintained in the predetermined range. The supplying rate of phosgene is determined depending on the rate of heat removal from the reactor because of the exothermic reaction. Further, in the case where the reaction mixture obtained by reacting alkali metal-tert-butoxide with carbon dioxide is successively used as a starting material of the reaction, the tertiary amine may be added at the time of the preceding reaction.

After the completion of the reaction, it is necessary in general to remove the residual phosgene from the reaction mixture by conventional means such as introducing an inert gas thereinto or distilling off a part of the solvent. Subsequently, it is also necessary to separate the crystals of alkali chloride which is the by-product salt precipitated in the mixture. This separating operation can be carried out by filtration. However, it is usually preferred to remove the alkali chloride by washing the mixture with water since the mixture shows a paste-like state as a whole. In this case, when the organic solvent used in the reaction is water-insoluble, the washing with water may be carried out by admixing water directly with the mixture. Whereas, when the organic solvent is water-soluble, it is desirable to wash the mixture with water after substituting the greater part of the solvent by a water-insoluble solvent such as dibutyl ether and heptane. There is thus obtained a solution of Di-BOC dissolved in the organic solvent free from alkali chloride as well as another impurities which are removed by washing with water. Then, Di-BOC can be recovered from the solution by a distillation under reduced pressure Lower distillation temperatures for recovering Di-BOC requires to reduce the pressure remarkably so that high cost for equipment is required. Whereas, higher distillation temperatures tend to increase a thermal decomposition of Di-BOC during distillation. Accordingly, the distillation temperatures are usually from 60° to 120°C, preferably from 60° to 100°C. It is desirable to conduct the distillation in the presence of a petroleum hydrocarbon having a boiling point of not lower than

250°C and a melting point of not higher than 20°C, in order to prevent the thermal decomposition of Di-BOC. Typical examples of such petroleum hydrocarbon are liquid paraffins such as ISOVG10, ISOVG15, ISOVG32, ISOVG68 and ISOVG100, which are defined by JIS-K2231 (1983). In this distillation, the petroleum hydrocarbon remains in the bottom of the distillation column, while Di-BOC is obtained from the top thereof. The amount of the petroleum hydrocarbon is usually from 0.01 to 10 times, preferably from 0.03 to 7 times, as much as the weight of Di-BOC which is supplied to the distillation system.

According to the present invention, it is possible to obtain Di-BOC in high yield directly by one-step reaction using monoalkali metal nono-tert-butyl carbonate as a starting material.

Consequently, the present invention provides a remarkably advantageous method on an industrial scale because the method is the shorter reaction step and does not need the purification of the intermediate i.e. tricarbonate as described in the known method. Additionally, the crystal of by-product i.e. alkali chloride can be removed from the reaction mixture containing Di-BOC which is stable to water by washing with water, whereby Di-BOC can be readily isolated.

Now, the present invention will be described in further detail with reference to Examples. However, it should be understood that the present invention is by no means restricted to such specific Examples.

EXAMPLE 1

Into a 3 ℓ glass reactor provided with a stirrer, a gas-inlet tube and a cooling jacket, 168 g (1.5 mol) of potassium tert-butoxide and 2060 ml of tetrahydrofuran were charged. Then 2 g (0.018 mol) of 1,4-diazabicyclo [2,2,2] octane was added thereto and 132 g (67.2 ℓ) of carbon dioxide was introduced into the mixture thus resulted over a period of 3 hours while maintaining the temperature of the reaction mixture to be 5°C under stirring to prepare mono-potassium mono-tert-butyl carbonate.
The reaction mixture thus obtained in this reaction was a pastlike slurry and mono-potassium mono-tert-butyl carbonate was formed quantitatively.
Subsequently, 223 g (2.25 mol) of phosgene was introduced into the said reaction mixture over a period of 4 hours while maintaining the internal temperature thereof to be 5°C under stirring to produce Di-BOC.

After the completion of the reaction, dibutyl ether in total amount of 2000 ml was added continuously into the reaction mixture while maintaining the temperature of not higher than 50°C under reduced pressure and distilling off tetrahydrofuran until the liquid height of the reaction mixture reached approximately at the same level as at the initial stage. The mixture was then cooled to 30°C and was washed twice with each 500 ml of water, followed by adding magnesium sulfate to the resultant organic layer to be dried. Subsequently, the greater part of dibutyl ether was distilled off under reduced pressure and further distillation was conducted at 62° - 66°C at 0.5 mmHg to recover 149 g of Di-BOC.

The yield of Di-BOC thus obtained based on monopotassium mono-tert-butyl carbonate and the purity were measured by liquid chromatographic analysis. The results are shown in Table 1.

EXAMPLES 2 TO 5

The reaction was conducted in the same manner as in Example 1 except that the tertiary amines and the reaction solvents were varied as described in Table 1. The yields and purities of Di-BOC are shown in Table 1.

EXAMPLES 6 and 7

The reaction was conducted in the same manner as in Example 1 except that the amounts of phosgene and the tertiary amine were varied. Namely, 80 g (0.81 mol) of phosgene was used and the used amount of 1,4-diazabicyclo [2,2,2] octane was shown in Table 1. The yields and purities of Di-BOC are shown in Table 1.

TABLE 1

| | Tertiary amine | | | Di-BOC | |
|---|---|---|---|---|---|
| | Compound | The amount used (mol) | Reaction solvent | Yield (%) | Purity (%) |
| Example | | | | | |
| 1 | 1,4-diazabicyclo [2,2,2] octane | 0.018 | Tetrahydrofuran | 90 | 98.5 |
| 2 | -do- | 0.005 | -do- | 88 | 98 |
| 3 | -do- | 0.018 | Dibutyl ether | 70 | 98 |
| 4 | -do- | -do- | Dioxane | 80 | 98 |
| 5 | Triethylamine | 0.200 | Tetrahydrofuran | 80 | 98 |
| 6 | 1,4-diazabicyclo [2,2,2] octane | 0.002 | -do- | 85 | 98 |
| 7 | -do- | 0.0002 | -do- | 80 | 98 |

COMPARATIVE EXAMPLE 1

The reaction was conducted in the same manner as in Example 1 except that no 1,4-diazabicyclo [2,2,2] octane, i.e. a tertiary amine was added, whereby there was scarcely obtained Di-BOC.

Further, although 1,4-diazabicyclo [2,2,2] octane was directly added in same amount as in Example 1 to the said reaction mixture obtained above and the reaction was continued, there was also scarcely obtained Di-BOC.

COMPARATIVE EXAMPLE 2

The operation was conducted in the same manner as in Comparative Example 1 except that the mixture obtained after introduction of phosgene was subjected to filtration over a period of 10 hours under reduced pressure and 1,4-diazabicyclo [2,2,2] octane was added in same amount as in Example 1 to the filtrate thus obtained followed to carry out the reaction at 25°C for 60 minutes. As a result, there was scarcely obtained Di-BOC.

EXAMPLE 8 TO 10

The operation was carried out in the same manner as in Example 1 except that a liquid paraffin (ISOVG32) was added in an amount as described in Table 2 to the crude Di-BOC, obtained by distilling off the greater part of dibutyl ether and then the resultant mixture was distilled to give pure Di-BOC at 85° - 90°C at 2 mmHg over a period of 6 hours.

The purity, the recovery rate and the decomposition rate of Di-BOC were measured. The results are shown in Table 2.

TABLE 2

| | Amount used of liquid paraffin (weight ratio)* | Distilled Di-BOC | | Decomposition Rate of Di-BOC (%) |
|---|---|---|---|---|
| | | Purity (%) | Recovery (%) | |
| Example | | | | |
| 8 | 0.2 | 98.5 | 94 | 3 |
| 9 | 0.1 | 98.5 | 94 | 4 |
| 10 | 0 | 97.0 | 80 | 15 |

* Weight ratio based on Di-BOC which was supplied to the distillation system

**Claims**

1. A process for producing di-tert-butyl dicarbonate which comprises reacting a monoalkali metal mono-tert-butyl carbonate with phosgene or trichloromethyl chloroformate in the presence of a tertiary amine in an organic solvent.

2. The process according to Claim 1, wherein the reaction temperature is maintained in the range of from -20° to 50°C.

3. The process according to Claim 1, wherein the amount of the tertiary amine is from 0.005 to 50 mol% per mol of the monoalkali metal mono-tert-butyl carbonate.

4. The process according to Claim 1, wherein the amount of phosgene or trichloromethyl chloroformate is from 0.4 to 5 mol per mol of the monoalkali metal mono-tert-butyl carbonate.

5. The process according to Claim 1, wherein the tertiary amine is at least one selected from the group consisting of an aliphatic amine and an alicyclic amine.

6. The process according to Claim 1, wherein the organic solvent is at least one selected from the group consisting of a cyclic ether and an aliphatic ether.

7. The process according to Claim 5, wherein the tertiary amine is 1,4-diazabicyclo [2,2,2] octane.

8. The process according to Claim 1, wherein the amount of the organic solvent is from 2 to 100 times as much as the weight of the monoalkali metal mono-tert-butyl carbonate.

9. The process according to Claim 4, wherein the amount of phosgene or trichloromethyl chloroformate is from 0.5 to 1.5 mol per mol of the monoalkali metal mono-tert-butyl carbonate.

10. The process according to Claim 3, wherein the amount of the tertiary amine is from 0.005 to 5 mol% per mol of the monoalkali metal mono-tert-butyl carbonate.

11. The process according to Claim 1, wherein the reaction time is from 0.5 to 20 hours.

12. The process according to Claim 1, wherein the monoalkali metal mono-tert-butyl carbonate is potassium salt.

13. The process according to Claim 1, wherein monoalkali mono-tert-butyl carbonate is obtained by reacting an alkali metal tert-butoxide with carbon dioxide at a temperature of from -20° to 30°C in an organic solvent.

14. The process according to Claim 1, wherein an alkali metal tert-butoxide is reacted with carbon dioxide at a temperature of from -20° to 30°C in an organic solvent and a resultant mixture containing monoalkali metal mono-tert-butyl carbonate is successively reacted with phosgene or trichloromethyl chloroformate in the presence of a tertiary amine.

15. The process according to Claim 1, wherein the reaction mixture containing di-tert-butyl dicarbonate is washed with water in the presence of a water-insoluble organic solvent to remove a by-product alkali chloride and after distilling off the water-insoluble organic solvent, di-tert-butyl dicarbonate is recovered by a distillation under reduced pressure.

16. The process according to Claim 15, wherein the distillation of di-tert-butyl carbonate is carried out in the presence of, a petroleum hydrocarbon having a boiling point of not lower than 250°C and a melting point of not higher than 20°C in an amount of from 0.01 to 10 times as much as the weight of di-tert-butyl carbonate which is supplied to the distillation system.

17. The process according to Claim 16, wherein the petroleum hydrocarbon is a liquid paraffin.